# NEUE EUROPÄISCHE PATENTSCHRIFT

(11) **EP 0 906 771 B2**
(45) Veröffentlichungstag und Bekanntmachung des Hinweises auf die Entscheidung über den Einspruch: **02.08.2006**
(45) Hinweis auf die Patenterteilung: 19.02.2003
(21) Anmeldenummer: 98118215.7
(22) Anmeldetag: 06.05.1996
(51) Int. Cl.: A61N 5/06, A61F 11/00

(54) **Therapiegerät mit einer Laserbestrahlungsvorrichtung**
Therapy apparatus with device for laser irradiation
Appareil de thérapie associé à un dispositif d'irradiation par laser

(30) Priorität: 16.05.1995 DE 29508077 U; 29.05.1995 DE 29508844 U; 20.09.1995 DE 29515096 U; 08.12.1995 DE 29519481 U; 08.12.1995 DE 29519482 U; 27.12.1995 DE 29520581 U
(43) Veröffentlichungstag der Anmeldung: 07.04.1999
(62) Teilanmeldung aus: 96914847.7
(73) Patentinhaber: Wilden, Lutz, 94051 Hauzenberg (DE)
(72) Erfinder: Wilden, Lutz, 94051 Hauzenberg (DE)
(74) Vertreter: Graf von Stosch, Andreas

(56) Entgegenhaltungen:
- EP-A- 0 537 385
- DE-C- 3 027 791
- DE-U- 9 402 306
- FR-A- 2 617 723
- US-A- 4 232 678
- US-A- 4 622 967
- US-A- 4 984 579
- "Tinnitus lindern durch Laserlicht". Dr.med. Lutz Wilden et al., Dr. Werner Jopp Verlag, Wiesbaden, 1994, ISBN 3-926955-69-4

## Beschreibung

Die vorliegende Erfindung bezieht sich allgemein auf Therapiegeräte mit einer Laserbestnihlungsvorrichtung. Insbesondere bezieht sich die Erfindung auf ein Innenohrstörungs-Behandlungsgerät zur Therapie einer chronischen komplexen Innenohrstörung.

Es werden von der Erfindung verwendete Low-Level-Laserbestrahlungsvorrichtungen bereits in zahlreichen Arztpraxen zur Behandlung von erkranktem Gewebe eingesetzt. Low-Level-Laserbestrahlungsvorrichtungen stellen somit ein bewährtes Behandlungsinstrument dar, so daß das erfindungsgemäße Gerät auf die hiermit gewonnenen Erfahrungen zurückgreifen kann, was dazu führt daß das erfindungsgemäße Gerät trotz äußerst geringem Aufwand mit sehr hoher Zuverlässigkeit ausgestattet werden kann; insbesondere ist es möglich, die Herstellungs- und Entwicklungskosten In vergleichsweise niedrigen Grenzen zu halten.

Der sogenannte Tinnitus stellt eine chronische komplexe Innenohrstörung dar. Es handelt sich hierbei um eine Erkrankung der Hörschnecke (Cochlea). Der sogenannte chronische vestibuläre Vertigo stellt demgegenüber eine Erkrankung des Vestibularorgans (des Labyrinths) des Ohres dar. Beide Erkrankungen sind häuflge Störungen des Innenohres und werden mit dem erfindungsgemäß Gerät vorzugsweise behandelt; jedoch können mit diesem Gerät ggf. auch weltere, hier nicht näher erläuterte Erkrankungen des Innenohres behandelt werden, wie z.B. die Innenohrschwerhörigkeit.

Für die betroffene Person äußert sich der Tinnitus als permanenter Pfeifton bzw. als ein ununterbrochenes Summgeräusch in bestimmten Frequenzen. Dieser permanente Pfeifton ist für die betroffene Person einerseits äußerstunangenehm und kann sogarzu psychischen Störungen führen, während andererseits das Hörvermögen In dem zugeordneten Frequenzbereich entsprechend eingeschränkt ist. Aus diesem Grund werden In letzter Zelt vermehrt Anstrengungen unternommen, den Tinnitus geelgneten Therapien zu unterziehen.

Als eine der erfolgreichsten Therapien zur Tinnitüsbehandlung hat sich in letzter Zelt ebenfalls die Verwendung eines Low-Level-Lasers herauskristallisiert. Im Falle der Tinnitusbehandlung mittels eines derartigen Low-Level-Lasers wurden insbesondere bei der Bestrahlung über das Mastoid (an einer ca. 2 cm hinter der Ohrmuschel befindlichen Stelle) oder des Gehörgangs bereits beachtliche Heilungserfolge erzielt. Auch der Vertigo und die Innenohrschwerhörigkeitkönnen mit dieser Methode behandelt werden.

Die genannten Low-Level-Laservorrichtungen sind Spezialgeräte, die derzeit nur in entsprechend eingerichteten Arztpraxen oder Kliniken vorhanden sind. Der betroffene Patient muß daher für jede innenohrstörungs- bzw. Tinnitusbehandlung eine solche Arztpraxis bzw. Klinik aufsuchen. Da eine fühlbare Heilungswirkung bel einer Low-Level-Laserbestrahlung in der Regel erst nach vergleichsweise langen Zeiträumen eintritt, muß der Patient entsprechend häufig die Arztpraxis bzw. Klinik aufsuchen. Dies ist einerselts für den Patienten lästig und hat andererseits den Nachteil, daß entsprechend hohe Behandlungskosten auftreten.

Aus der US-A-4 232 678 ist eine Vorrichtung zur lokalen Behandlung eines Patienten durch Akupunktur oder Aurikulotherapie bekannt, bei welcher vorbestimmte Bereiche des Körpers des Patienten mit Licht aus einer Laserdiode beaufschlagt werden. Aus der DE 9402306 01 ist bekannt, einen laser zur Behandlung von Tinnitus einzusetzen. Dieser Laser ist über einen Licht-leiter mit einem Lasermitter verbunden, wobei der laser eine separate, stationäre Einheit darstellt.

Der Erfindung liegt die Aufgabe zugrunde, ein Innenohrstörungs-Behandlungsgerät zu schaffen, mit dem die Behandlungskosten deutlich herabgesetzt werden können.

Diese Aufgabe wird erfindungsgemäß mit der im Anspruch angegebenen Maßnahme gelöst.

Die Erfindung schlägt in diesem Zusammenhang vor, eine Low-Level-Laserbestrahlungsvorrichtung vorzusehen, die in einer geeigneten Befestigungsvorrichtung lösbar derart am Ohr eines Patienten befestigt werden kann, daß der Laserstrahl auf mindestens einen vorbestimmten Bereich des Ohres einwirkt. Der Kemgedanke dieser Maßnahme ist somit darin zu sehen, eine Low-Level-Laserbestrahlungsvorrichtung zu schaffen, die der Patient für die Dauer der Behandlung ständig mit sich trägt, so daß eine entsprechend Intensive Behandlung erfolgt, was erwarten läßt, daß die Heilungsaussichten mit dem erfindungsgemäßen Gerät sogar noch höher sind, als mit den in Arztpraxen bzw. Kliniken vorhandenen herkömmlichen Low-Level-Laservorrichtüngen. Ein weiterer Vorteil des erfindungsgemäßen Innenohrst6rungs-Behandlungsgeräts liegt darin, daß es im Prinzip ausreicht, wenn das Gerät von einem entsprechenden Therapeuten zu Beginn der Behandlung angepaßt wird; weitere Arztbesuche sind für den Patienten dann nicht mehr notwendig, so daß die Therapie für den Patienten entsprechend bequemer ist. Die mit den Arzt-bzw. Klinlkbesuchen verbundenen Behandlungskosten entfallen ebenfalls, so daß Insgesamt große Kostenvortelle erzielt werden.

Die Low-Level-Laserbestrahlungsvorrichtung des erfindungsgemäßen Innenohrstörungs-Behandlungsgeräts ist in der Befestigungsvorrichtung selbst untergebracht, wobei in diesem Fall vorzugsweise eine Lichtleitervorrichtung vorgesehen ist, über die der von der Laserbestrahlungsvorrichtung abgegebene. Laserstrahl ggf. einer Linse zugeführt wird, aus der der Laserstrahl austritt und auf den/die vorbestimmten Bereich (e) einwirkt. Der Lichtleiter besteht vorzugsweise aus einem Material, das es dem das Gerät anpassenden Therapeuten gestattet, die Position des Lichtaustritts, d.h. den vorbestimmten Wirkungsbereich, durch Verbiegen, Verschwenken oder dergleichen einzustellen. Gegebenenfalls kann zwischen der Befestigungsvorrichtung und der Lichtleitervorrichtung auch eine Verstellvorrichtung vorgesehen werden, die es gestattet, die Länge und/oder Richtung der Lichtleitervorrichtung durch Verschrauben oder dergleichen zu ändem.

Ein weiterer, wesentlicher Aspekt bei diesem Gerät liegt darin, daß als Befestigungsvorrichtung eine bereits vorhandene Einrichtung verwendet wird. Bei einer solchen Einrichtung kann es sich insbesondere um ein Brillengestell, ein Hörgerät, einen Tinnitusmasker oder um ein Kombinationsgerät aus einem Tinnitusmasker und einem Hörgerät handeln (ein Tinnitusmasker ist ein kleiner Lautsprecher, der ein Geräusch in der Frequenz des Tinnitus erzeugt, so daß der Patient den Eindruck hat, beim Tinnitus würde es sich um ein extemes Geräusch handeln). Selbstverständlich ist es jedoch auch möglich, für das erfindungsgemäße Innenohrstörungs-Behandlungsgerät eine eigene Befestigungsvorrichtung vorzusehen, wie beispielsweise einen am Ohr befestigbaren Bügel, ein in das Ohr einführbares Teil (vergleichbar einem Innenohr-Hörgerät) oder einen auf den Kopf aufstülpbaren Bügel nach Art eines Kopfhörers.

Untersuchungen haben gezeigt, daß mit den erfindungsgemäßen Geräten insbesondere dann große Erfolge erzielbar sind, wenn die Low-Level-Laserbestrahlungsvorrichtung einen Laserstrahl mit einer Wellenlänge erzeugt, die sich vom Ultraviolettbereich bis hin zum nahen infrarotbereich erstreckt, also von circa 180 nm bis etwa 1000 nm, wobei die Ausgangsleistung bei den zur Eigenbehandlung vorgesehenen Geräten vorzugsweise zwischen 1 mW und 120 mW, insbesondere zwischen 1 mW und 5 mW liegen soitte. Wenn die Geräte demgegenüber für den professionellen Gebrauch durch geschufte Therapeuten bestimmt sind, werden Laserbestrahtungsvorrichtungen mit Ausgangsleistungen bis maximal 500 mW eingesetzt.

Gegebenenfalls kann daran gedacht werden, das Gerät mit einer Hand-Einstellvorrichtung zu versehen, mittels der die Ausgangsleistung der Low-Level-Laserbestrahlungsvorrichtung und/oder die Wellenlänge des Laserstrahls vom Patienten auf einen wählbaren Wert eingestellt werden kann. Die Low-Level-Laserbestrahlungsvorrichtung kann entweder im kontinuierlichen Betrieb oder auch im pulsierenden Betrieb arbeiten, wobei die gewünschte Arbeitsweise gegebenenfalls mit einem entsprechenden Wählschaltereingestellt werden kann.

Die Erfindung wird nachstehend anhand der Beschreibung von Ausführungsbeispielen unter Bezugnahme auf die Zeichnung näher erläutert Es zeigen:

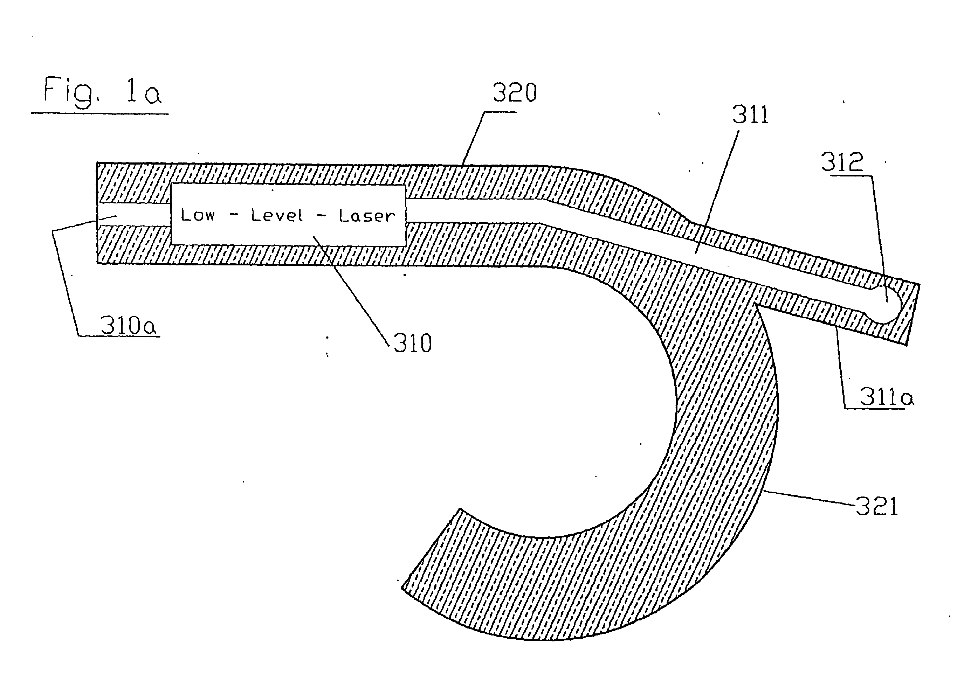

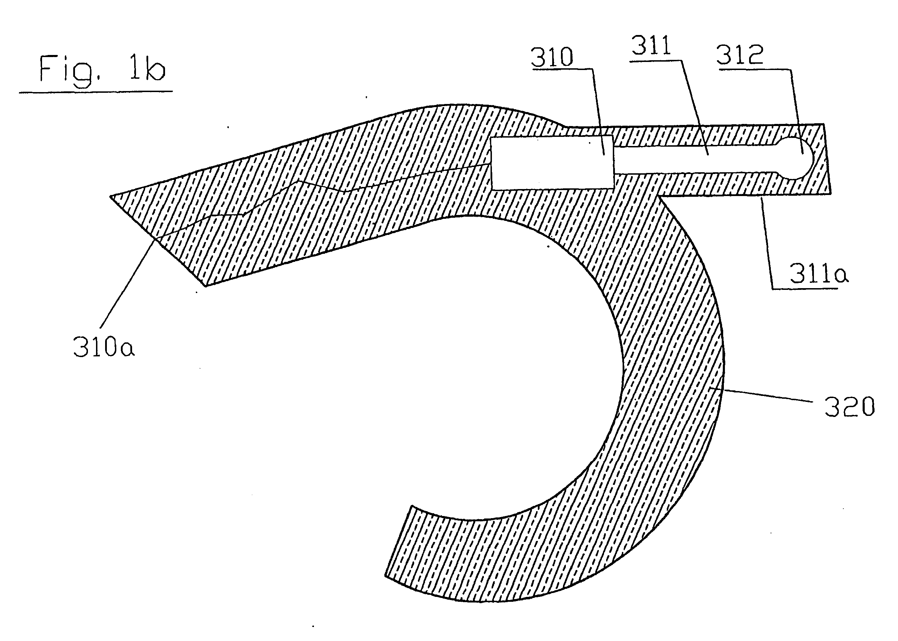

Fig.1A und 1B zwei Ausführungsformen eines Innenohrstörungs-Behandiungsgeräts zur Therapie einer chronischen komplexen Innenohrstörung:
   Gemäß Fig.1A weist eine erste Ausführungsform des erfindungsgemäßen Innenohrstörungs-Behandlungsgeräts eine Low-Level-Laserbestrahlungsvorrichlung 310 auf, die In das Innere eines Brillengestells 320 In der Nähe des Bügels 321 desselben eingebaut ist. Die Laserbestrahlungsvorrichtung 310 wird über Kabel 310a aus einer (nicht gezeigten) Stromquelle in Form einer Batterie oder dergleichen gespeist. Der von der Laserbestrahlungsvorrichtung 310 abgegebene Laserstrahl wird über eine Lichtleitervorrichtung 311 einer Linse 312 zugeführt, aus der er austritt und auf den darunter liegenden Bereich einwirkt. Die Lichtleftervorrichtung 311 verläuft austrittsseitig in einem etwa rohrförmigen Ansatz 311 a, der aus einem elastisch verformbaren Material besteht, so daß die Position der Linse 312 und damit der Wirkbereich des erfindungsgemäBen Innenohrstörungs-Behandlungsgeräts geändert bzw. an den Patienten angepaßt werden kann. Da diese erste Ausführungsform in eine Brille integriert ist, wird somit ein Kombinationsgerät geschaffen; das bei einem Brillenträger kaum weiter auffällt.
   In Fig.1 B ist eine weitere Ausführungsform des erfindungsgemäßen Innenohrstörungs-Behandlungsgeräts gezeigt, das sich von der ersten Ausführungsform dadurch unterscheidet, daß es sich bel der Befestigungsvorrichtung 320 um einen Bügel handelt, der beispielsweise ein speziell für das Innenohrstörungs-Behandlungsgerät gefertigtes Teil darstellt. Der Bügel 320 kann aber auch Teil eines Hörgeräts, eines Tinnitusmaskers oder eines Kombinationsqeräts aus einem Hörgerät und einem Tinnitusmasker sein. Im übrigen entspricht diese zweite Ausführungsform des innenohrstörungs-Behandlungsgeräts dervorstehend beschriebenen ersten Ausführungsform, so daß bezüglich seiner technischen Einzelheiten auf die vorstehende Beschreibung verwiesen werden darf.

Für die Low-Level-Laserbestrahlungsvorrichtung 310 kann ein (in den Figuren nicht gezeigter) Ein/Aus-Schalter vorgesehen werden, der es dem Patienten ermöglicht, die Behandlung zu bellebigen Zeiten vorzunehmen. Die Laserbestrahlungsvorrichtung 310 kann weiterhin eine manuelle Einstellvorrichtung aufweisen, mittels der Ihre Ausgangsleistung und/oder die Wellenlänge des Laserstrahls auf einen geeigneten Wert eingestellt werden kann. Die Laserbestrahlungsvorrichtung 10 arbeitet entweder Im kontinuierlichen oder im pulsierenden Betrieb, wobei gegebenenfalls eine Steuervorrichtung vorgesehen sein kann, mittels der die gewünschte Betriebsart und/oder die Impulsfrequenz eingestellt werden kann. Die Ausgangsleistung der Laserbestrahlungsvorrichtung 310 beträgtvorzugsweise zwischen 1 und 120 mW. Die Wellenlänge des Laserstrahls liegt im Bereich von 180 nm bis 1000 nm. Somit ist es möglich, handelsübliche Laserdioden als Strahlungsquelle zu verwenden.

Untersuchungen haben gezeigt, daß mit dem erfindungsgemäßen Gerät Insbesondere dann gute Hellungserfolge erzielbar sind, wenn der Laserstrahl auf das Mastoid oderüber den Gehörgang auf das Mittelohr einwirkt. Diese Bereiche des Ohres können durch geeignete Einstellung der Lichtleltervorrichtung 311 bestrahlt werden. Um eine noch bessere Heilungswirkung zu erzielen, kann das erfindungsgemäße Gerät gegebenenfalls dahingehend modifiziert werden, daß zwei Lichtleüervorrichtungen mit einer jeweiligen Linse vorgesehen werden, wobei die eine Linse auf das Mastoid und die andere Linse auf das Mittelohr einwirkt. Die Anzahl der Lichtleitervorrichtungen und Linsen kann selbstverständlich weiter erhöht werden, falls je nach der Art der Erkrankung des Innenohrs noch andere Bereiche des Ohres therapeutisch beaufschlagt werden sollen.

## Patentansprüche

1. Innenohrstörungs-Behandlungsgerät zur Therapie einer chronischen komplexen Innenohrstörung eines Patienten, insbesondere zur Behandlung von Tinnitus und Vertigo, umfassend
eine Low-Level-Laserbestrahlungsvorrichtung (310) und eine Befestigungsvorrichtung (320) mittels derer die Low-Level-Laserbestrahlungsvorrichtung lösbar derart am Ohr eines Patienten befestigbar ist, dass der Laserstrahl auf mindestens einen vorbestimmten Bereich des Ohres einwirkt, wobei die Low-Level-Laserbestrahlungsvor-richtung (310), umfassend einen Low-Level-Laser, in der Befestigungsvorrichtung (320) selbst untergebracht ist.

2. Innenohrstörungs-Behandlungsgerät nach Anspruch 1, **dadurch gekennzeichnet, dass** die Low-Level Laserbestrahlungsvorrichtung (310) auf das Mastoid und/oder über den Gehörgang auf das Mittelohr einwirkt.

3. Innenohrstörungs-Behandlungsgerät nach Anspruch 1 oder 2, **dadurch gekennzeichnet, dass** die Low-Level-Laserbesttahlungsvorrichtung (310) auf den/die vorbestimmten Bereich(e) des Ohres über eine jeweilige Lichtleitervorrichtung (311) und eine an deren Ende sitzende Linse (312) einwirkt.

4. Innenohrstörungs-Behandlungsgerät nach einem der Ansprüche 1 bis 3, **dadurch gekennzeichnet, dass** die Befestigungsvorrichtung (320) ein Brillengestell, ein Hörgerät, ein Tinnitusmasker oder ein Kombinationsgerät aus einem Tinnitus-masker und einem Hörgerät ist.

5. Innenohrstörungs-Behandlungsgerät nach einem der Ansprüche 1 bis 4, **dadurch gekennzeichnet, dass** die Befestigungsvorrichtung (320) ein am Ohr befestigbater Bügel, ein in das Obt einführbares Teil oder ein auf den Kopf aufstülpbaret Bügel ist.

## Claims

1. Inner-ear disorder treatment apparatus for therapy of a chronic complex inner ear disorder of a patient, particularly for treatment of tinnitus and vertigo, comprising
a low-level laser rirradiation device (310) and an attachment device (320) by means of which the low-level laser irradiation device can be releasably attached to the ear of a patient in such a way that the laser beam acts on at least one predetermined region of the ear, whereby the low-level laser irradiation device (310) comprising the low-level laser is housed in the attachement device (320).

2. Inner-ear disorder treatment apparatus according to claim 1, **characterized in that** the low-level laser irradiation device (310) acts on the mastoid and/or via the auditory canal on the middle ear.

3. Inner-ear disorder treatment apparatus according to claim 1 or 2, charcterized in that the low-level laser irridation device (310) acts on the predetermined region(s) of the ear via a light-guide device (311) and a lens located at its end (312) in each instance.

4. Inner-ear disorder treatment apparatus according to one of claims 1 to 3, **characterized in that** the attachment device (320) is a glasses frame, a hearing aid, a tinnitus masker, or a combination apparatus consisting of a tinnitus masker and a hearing aid.

5. Inner-ear disorder treatment apparatus according to one of claims 1 to 4, **characterized in that** the attachment device (320) is an earpiece which can be attached to the ear, a part which can be inserted into the ear, or a strap which can be placed on the head.

## Revendications

1. Appareil pour le traitement des troubles de l'oreille interne, destiné à la thérapie des troubles complexes chroniques de l'oreille interne d'un patient, en particulier au traitement des acouphènes et des vertiges, comprenant :
un dispositif d'irradiation par laser faible puissance (310) et un dispositif de fixation (320) au moyen duquel le dispositif d'irradiation par laser faible puissance peut être mis en place de façon amovible sur l'oreille d'un patient, de telle manière que le rayon laser exerce ses effets sur au moins une zone prédéterminée de l'oreille, dans lequel le dispositif de traitement par laser faible puissance (310), comprenant un laser faible puissance, est implanté lui-même dans le dispositif de fixation (320).

2. Appareil pour le traitement des troubles de l'oreille interne selon la revendication 1, **caractérisé en ce que** le dispositif d'irradiation par laser faible puissance (310) exerce ses effets sur l'apophyse mastoïdienne et/ou sur l'oreille interne par le biais du conduit auditif.

3. Appareil pour le traitement des troubles de l'oreille interne selon la revendication 1 ou 2, **caractérisé en ce que** le dispositif d'irradiation par laser faible puissance (310) exerce ses effets sur la ou les zones prédéterminées de l'oreille par l'intermédiaire d'un dispositif guide de lumière correspondant (311) et une lentille (312) à son extrémité.

4. Appareil pour le traitement des troubles de l'oreille interne selon l'une des revendications 1 à 3, **caractérisé en ce que** le dispositif de fixation (320) est une monture de lunettes, un appareil auditif, un masqueur d'acouphènes ou un appareil combinant un masqueur d'acouphènes et un appareil auditif.

5. Appareil pour le traitement des troubles de l'oreille interne selon l'une des revendications 1 à 4, **caractérisé en ce que** le dispositif de fixation (320) est un étrier pouvant être fixé sur l'oreille, une pièce pouvant être introduite dans l'oreille ou une monture pouvant être mise en place sur la tête à la manière d'un serre-tête.
